(19) Europäisches Patentamt

European Patent Office

Office européen des brevets

(11) EP 1 736 134 A1

## (12) EUROPEAN PATENT APPLICATION

(43) Date of publication:
27.12.2006 Bulletin 2006/52

(51) Int Cl.:
*A61K 8/02* (2006.01)    *A61K 8/11* (2006.01)
*A61Q 1/00* (2006.01)

(21) Application number: 06253170.2

(22) Date of filing: 20.06.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 22.06.2005 JP 2005182696
25.11.2005 JP 2005340979

(71) Applicant: L'Oreal
75008 Paris (FR)

(72) Inventor: Ikkai, Fumiyoshi
Takatsu-ku
Kawasaki,
Kanagawa, 213-0012 (JP)

(74) Representative: McCluskie, Gail Wilson
J.A. Kemp & Co.,
14 South Square,
Gray's Inn
London WC1R 5JJ (GB)

### (54) Spherical optical structure

(57) A cosmetic coloring material comprising the spherical optical structure and a cosmetic composition containing the coloring material are disclosed. The spherical optical structure has a particle diameter of 500 μm or less, comprising a core substance and a coating substance coating the core substance, wherein the particle diameter and the thickness of the coating substance of the spherical optical structure are controlled to be in a predetermined range, according to the refractive index of an external medium in contact with the outer surface of the spherical optical structure and the refractive index of the coating substance, so as to display a structural color in the visible light range.

EP 1 736 134 A1

**Description**

[Technical Area]

The present invention concerns a spherical optical structure capable of displaying a structural color, and cosmetic uses thereof.

In the field of cosmetic products, pigments, colorants, dyes, and the like are widely used as coloring materials for coloring a cosmetic ingredient. Coloring has been carried out by mixing a specific quantity of these coloring materials into the cosmetic ingredient composition, so that the cosmetic ingredient being employed becomes affixed with pigment or colorant, or dyed with a dye. In the case of such conventional coloring materials as these pigments, colorants, or dyes, differences occur in the wavelength characteristics of the light reflected from the surface of the coloring material due to differences in the wavelength-dependency of absorbance, i.e., due to differences in the absorption spectrum characteristics, at the surface of the coloring material. As a result, an observer will be cognizant of coloring differences.

In the case of coloring materials such as described above, mixing of a plurality of coloring materials has generally been attempted in order to obtain variety in coloring. In addition, in the case of use as a cosmetic ingredient, additives such as inorganic layered composite powders like mica, pearlescent pigments, liquid-crystal compounds, and the like are mixed into the composition used for the cosmetic, for the purpose of adding gloss or luster to the surface of the skin, nails or hair.

For example, a color pearling agent, in which a colored powder such as iron oxide, smalt, chromium hydroxide or carmine is mixed with or coated onto titanium mica, which has a pearl luster and an interference color, is an ingredient that is essential as a lustrous coloring material in a cosmetic product.

[Non-Patent Document]

Development of Advanced Cosmetics, CMC Publishing Co., Ltd., 297-306 (2000).

[Disclosure of the Invention]

[Problems to be Solved by the Invention]

However, mixing of conventional coloring materials leads to a subtractive color mixture due to absorption of light by each of the respective coloring materials. As a result, it is not always possible to generate the unique characteristics of each of the coloring materials, and combining of coloring materials tends to result in a deterioration in color saturation.

Moreover, additives such as inorganic layered composite powders, pearlescent pigments, liquid-crystal compounds and the like are themselves colorless or white, or have little variety of color, so that they must be used in combination with other coloring materials in order to obtain the desired coloring. In this case, due to combination with a coloring material that has low color saturation and brightness, it is not always possible to produce the lustrous sensation required from these additives.

On the other hand, colored powders that are mixed into the aforementioned color pearling agent, which is a conventional combination deemed to have good color saturation, do not always have superior chemical stability. For example, smalt has poor alkali and thermal resistance, and carmine has poor light fastness.

Further, there are many conventional pigments, colorants, and dyes that, depending on the quantity employed, are not entirely harmless with respect to the human body, e.g., effect on skin.

Accordingly, the present invention employs a principle that differs from coloring methods using absorption of a portion of light as in the case of conventional coloring materials, and aims to provide a spherical optical structure for a non-toxic, chemically stable cosmetic composition that displays a bright structural color regardless of the direction of view. The present invention further aims to provide a cosmetic composition as described above.

As a method for creating mass amounts of minute spherical particles having a shell structure, the production method of microcapsules is known. Microcapsules comprise a core substance and a coating, and a wide variety of applications have been investigated. In particular, in the fields of cosmetics and pharmaceuticals, they have been used for improving the stability of the various effective components in a composition, and imparting sustained release properties, for the blocking of odors and tastes originating from the effective components, and the like. For example, blue coloring materials and the like wherein pigments or dyes are enclosed within microcapsules are known (see e.g., Japanese Patent Publication (Kohyo) No. 2004-526558). However, no attempt has ever been made until now of coloring by means of the microcapsules themselves, without the use of conventional pigments or dyes.

[Means for Solving the Problems]

The inventors of the present invention came upon the present invention by discovering that coloring in a desired structural color in the visible light range can be obtained, in a spherical optical structure having a particle diameter of 500 $\mu$m or less, comprising a core substance and a coating substance coating the core substance, by controlling the particle diameter and the thickness of the coating substance of the aforementioned spherical optical structure, according to the refractive index of the external mediumin contact with the outer surface of the aforementioned spherical optical structure, and the refractive index of the aforementioned coating substance.

Structural colors differ from pigment colors, which are based on the absorption of light by substances, in that they are colors that are created based upon the microstructure of substances, and this relates to the scattering and interference of light. In cosmetics, inorganic substance particles having a multilayered thin film structure are used as angle dependent coloring materials for which the color changes depending upon the viewing angle. However, no examples have been reported of a structural color having been obtained by adjusting the particle diameter or the thickness of the coating of spherical optical structures in the form of microcapsules.

The spherical optical structure of the present invention can develop a structural color according to the particle diameter and/or thickness of the coating material, without using conventional colorants such as pigments or dyes. The structural colors obtained have a unique transparent feel, and by blending this into various cosmetics, it becomes possible to obtain specific hues that were not possible conventionally.

Additionally, for the spherical optical structure of the present invention, the toxicity of the core substances (internal phase) can be suppressed by utilizing appropriate coating substance materials, such as polystyrene or the like, so the safety is high.

[Best Mode for Embodying the Invention]

It is sufficient for the core substance contained in the spherical optical structure of the present invention to be a material having a refractive index different from that of the coating substance, and it is preferable for it to comprises an aqueous or hydro-organic solution comprising water and/or C1-C6 alcohol such as ethanol and/or isopropanol; or a liquid substance such as a organic liquid substance and/or an inorganic solvent or the like, or a mixture thereof, or a semiliquid material having a certain degree of viscosity. For example, from the standpoint of ease of preparation, it is preferable that the core substance comprises aqueous or hydro-organic soluiton, and particularly that it comprises an aqueous solution wherein gelatin or hydrophilic thickening agents is dissolved. Additionally, the core substance may be air (the interior being hollow).

It is sufficient for the material constituting the coating to be a material conventionally used for the formation of microcapsules generally, and this is selected from among materials having a refractive index different from that of the medium wherein the spherical optical structures of the present invention are blended. For example, polymers such as vinyl based polymers such as polystyrene, polyalkylenes such as polyethylene; C1-C32 alkyle poly(meth)acrylate such as methyl poly(meth)acrylate; poly(meth)acrylamide; alkyl polyacetate such as ethyl polyacetate; and/or polycondensation polymers such as polycarbonate, polyurethane, nylon, polyester and the like; and/or cellulose based polymers such as cellulose acetate, ethylcellulose and the like; and/or silicone based polymers such as polyalkylsiloxane, polydimethylsiloxane and the like; and/or organic polymers such as chloride polymers, fluorine based polymers and the like; and/or inorganic materials such as glass, silica, titania, and the like.

The spherical optical structure of the present invention is a spherical optical structure with a particle diameter of 500 $\mu$m or less, comprising a core substance and a coating substance coating the core substance, characterized in that a structural color in the visible light range is displayed by controlling the particle diameter and the thickness of the coating substance of the aforementioned spherical optical structure to be in a predetermined range, according to the refractive index of the external medium in contact with the outer surface of the aforementioned spherical optical structure and the refractive index of the aforementioned coating substance.

Additionally, the spherical optical structure of the present invention is characterized by being a spherical optical structure having a particle diameter of 500 $\mu$m or less, comprising a core substance and a coating substance that coats the core substance, wherein by controlling the particle radius L and the thickness of the coating substance D of the aforementioned spherical optical structure according to the refractive index $n_1$ of the external medium in contact with the outer surface of the aforementioned spherical optical structure and the refractive index $n_2$ of the aforementioned coating substance, a structural color with the wavelength $\lambda$ in the visible light range obtained by substituting a value d satisfying

[Equation 1]

$$sin\{90 - \arccos(\frac{L-d}{L})\} = \frac{n_2}{n_1} sin[\{90 - \arccos(\frac{L-d}{L})\} - \arctan[\frac{D-d}{Lsin\{\arccos(\frac{L-d}{L})\}}]]$$

into

[Equation 2]

$$\frac{\lambda}{2}(2m+1) \ (m = 0, 1, 2, 3, .....) = \frac{2Lsin\{\arccos(\frac{L-d}{L})\}}{cos[\arctan[\frac{D-d}{Lsin\{\arccos(\frac{L-d}{L})\}}]]} - 2Lsin\{\arccos(\frac{L-d}{L})\}$$

(where m is 0 or a natural number) is displayed.

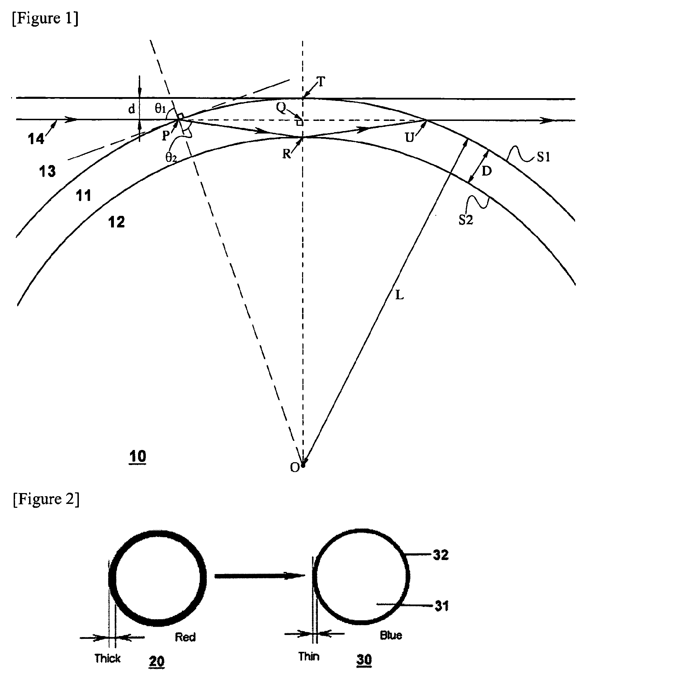

Figure 1 shows a diagram explaining the difference in the length of the light path, in comparison with directly propagating light, created by the geometrical relationship between the particle radius L of the spherical optical structure of the present invention, the thickness D of the coating substance, the coating substance (refractive index $n_2$), and the external medium in contact with the outer surface of the aforementioned spherical optical structure (refractive index $n_1$).

A light beam 14 enters through an external medium 13 (refractive index $n_1$) in contact with the outer surface of a spherical optical structure, is refracted according to Equation 1 derived from Snell's Law ($n_1 sin\theta_1 = n_2 sin\theta_2$) at point P on the coating outer wall S 1 that is the surface of the boundary with the coating 11 (refractive index $n_2$) of the spherical optical structure 10, and enters the internal portions of the coating 11. The incident light beam is reflected at point R on the coating inner wall S2 of the coating 11, and exits back into the external medium 13 from point U on the coating outer wall S1. At this time, at point U, interference occurs between the light beam 14 that has propagated through the path going through the abovementioned point P, point R, and point U, and light that has propagated through the external medium 13 through a distance corresponding to the straight line between point P and point U. In this case, for a wavelength λ that satisfies the left side of Equation 2 for the light path difference arising between the two light beams (corresponding to the right side of Equation 2), conditions arise wherein the light is strengthened due to constructive interference effects.

Therefore, an optimal spherical optical structure can be obtained for a wavelength λ for which coloring by a structural color is desired, by controlling the thickness D of the coating and the particle radius L of the spherical optical structure so as to simulatneously satisfy the aforementioned Equation 1 and Equation 2.

Since the m on the left side of equation 2 is 0 or a natural number, and the interference effects are strongest at m=0, the spherical optical structure of the invention of the present application is primarily designed for the condition m=0. However, colors for the conditions of m=1 or above may also be contained at the same time.

"Controlling the particle diameter and the thickness of the coating substance of the aforementioned spherical optical structure to be in a predetermined range, according to the refractive index of the external medium in contact with the outer surface of the aforementioned spherical optical structure and the refractive index of the aforementioned coating substance" means that, for a given combination of the refractive index of the external medium in contact with the outer surface of the spherical optical structure and the refractive index of the substance selected as a coating substance, the thickness of the coating substance is produced so as to be within a specific range necessary for displaying a desired structural color, for a given particle diameter of the spherical optical structure.

Further, for a given combination of the refractive index of the external medium in contact with the outer surface of the spherical optical structure and the refractive index of the substance selected as a coating substance, a plurality of pairs of combinations of the particle diameter of the spherical optical structure and the coating thickness exists, but it is sufficient for the dimensions of the produced spherical optical structures to be within a specific range that results in the development of the desired structural color.

In addition to the exact matching of the coating thickness to the thickness D that satisfies Equation 1 and Equation 2 whereby the wavelength λ of the desired structural color is derived, thicknesses whereby the structural coloring can be developed that roughly match the thickness D are also included. Further, in addition to cases wherein the light path difference created between a light beam that has passed through the coating of the spherical optical structure and direct light is equivalent to half of the wavelength (half-wavelength), cases where this is equivalent to odd multiples of half-wavelengths are also included.

The thickness D of the coating becomes smaller as the wavelength $\lambda$ of the structural color becomes shorter (bluer) and the difference in refractive index between the refractive index $n_1$ of the external medium and the refractive index of the coating substance $n_2$ becomes larger, whereas it becomes larger as the wavelength $\lambda$ of the structural color becomes longer (redder), and the difference in refractive index between the refractive index $n_1$ of the external medium and the refractive index of the coating substance $n_2$ becomes smaller. If all combinations of the external medium and the coating substance are considered, it is preferable for the coating thickness D to be in the range of 50 to 700 nm. In cases where the external medium is a medium used in cosmetics such as water, alcohol, air, or the like, and the coating substance is a high molecular polymer, the range 80 to 500 nm is preferable, and the range 100 to 300 nm is more preferable.

Since the particle radius L has a small effect on the wavelength $\lambda$ of the structural color in comparison with the coating thickness D, there are no restrictions on the particle diameter that are dependent on the wavelength of the structural color to be developed. However, if general conditions with regard to lustrous sensation or the depth of wrinkles in the skin or the like, upon use as a cosmetic coloring material, or a cosmetic composition containing this are taken into consideration, it is preferable for the diameter to be 500 $\mu$m or smaller, and if the production of the structures and the control conditions are also taken into consideration, a range of 1 to 500 $\mu$m is preferable, and a range of 10 to 300 $\mu$m is more preferable.

The spherical optical structures of the present invention can be produced according to methods used for the production of conventional microcapsules. For example, the interfacial polymerization method, the phase separation method, the interfacial precipitation method, the spray drying method, and the fluid bed method are known. However, the method of preparation is not restricted to conventional methods, and any method may be used. Preferably, the method should be suited to controlling the particle diameter and/or coating thickness of the spherical optical structures to be within a desired range.

For the spherical optical structure of the present invention, from the standpoint of ease of manipulation, it is preferable to use the interfacial precipitation method.

The interfacial precipitation method is a microencapsulation method that is also called the secondary emulsion method, the submerged concentration method, the submerged drying method, and the like (Microcapsules: Their Production, Properties, and Applications, Tamotsu Kondou et al., Sankyou Publishing). Concretely, this can be classified into an interfacial precipitation method in water using a water/oil/water type compound emulsion, and an interfacial precipitation method in oil using an oil/water/oil type compound emulsion, and selection can be done appropriately according to the properties of the materials to be utilized as the core substance and the coating substance.

If an aqueous solution of gelatin is used as the core substance, and a hydrophobic organic polymer such as polystyrene is used as the coating substance, it is preferable to use the interfacial precipitation method in water. This shall be explained in more detail as follows.

An oil/water emulsion (primary emulsion) is formed wherein an approximately 4 to 8 wt% gelatin aqueous solution is dispersed in an approximately 1 to 5 wt% methylene chloride solution of polystyrene. Next, said primary emulsion is dispersed in water to form a water/oil/water type secondary emulsion. Next, treatments such as heating, pressure reduction, solvent extraction, freezing, cooling, and dry powder treatment may be optionally performed to obtain the spherical optical structures.

The solvent for dissolving the coating substance (hydrophobic polymer) in the aforementioned interfacial precipitation method may be selected as appropriate according to the type of polymer, but examples are methylene chloride (in the case of polystyrene or polycarbonate), benzene (in the case of ethylcellulose), cyclohexane (in the case of ethylcellulose), and carbon tetrachloride (in the case of polystyrene).

In the present invention, by adjusting the concentration of coating substance when preparing the primary emulsion, the coating thickness was adjusted, and a spherical optical structure in the form of a microcapsule, that displays a desired structural color, was obtained.

For example, if an approximately 5 wt% polystyrene solution (methylene chloride) is used, a spherical optical structure having a thickness of 100 to 1000 nm can be obtained, and in a secondary emulsion wherein such spherical optical structures are dispersed in water, spherical optical structures developing a plurality of structural colors from red to blue existed together.

On the other hand, if an approximately 1 wt% polystyrene solution was used, the ratio of spherical optical structures displaying a blue structural color increased.

Additionally, the created secondary emulsion contained particles of various sizes (particle diameters), but the particle diameters of spherical optical structures displaying structural colors was approximately 1 to 200 $\mu$m. However, no correlation could be seen between the particle diameter of the spherical optical structures and the structural colors they displayed.

That is, for the spherical optical structures of the present invention, the greatest factor determining the structural colors thereof is thought to be the coating thickness. Therefore, as shown in figure 2, for example, if the other adjustable conditions were identical, the number of spherical optical structures displaying cold structural colors close to blue could be increased by decreasing the coating substance concentration in the solution, whereas the ratio of spherical optical structures displaying warm structural colors close to red could be increased if the coating substance concentration in the solution is not decreased.

Further, the particle diameter and/or coating thickness of the spherical optical structures can be controlled to be within a desired range by, for example, sifting using a differentiation means such as filtering or centrifuging on the rough structures produced by a conventional microcapsule preparation method or the like such as the interfacial precipitation method.

Spherical optical structures obtained in this way can be dried by performing a normally used treatment during the interfacial precipitation method, and used as solid particles, or used as dispersants within a medium obtained as a secondary emulsion.

The spherical optical structure of the present invention displays hues having a unique transparent sensation, and by mixing this into cosmetic compositions as a cosmetic coloring material, a unique chromatic effect not obtainable conventionally can be obtained.

Therefore, the present invention provides a cosmetic coloring material comprising the aforementioned spherical optical structures displaying structural colors, and a cosmetic composition containing said coloring material.

The cosmetic coloring material of the present invention may be in the form of dry particles or dispersed throughout a medium. Additionally, the cosmetic composition of the present invention may contain, in addition to the aforementioned spherical optical structures displaying structural colors, additives that are normally used in cosmetics, for example antioxidants, fragrances, essential oil, preservatives, cosmetic activator, vitamins, necessary fatty acids, sphingolipids, self tanning compounds such as DHA, and sunscreen agents.

The composition of the present invention is intended for applying to skin of the face and body, mucous membranes and/or keratin fibers such as nails, eyelashes, or hair.

These can be in the form of any cosmetics imaginable, for example, solid or flexible oil gels that may optionally contain water; oil in water type, or water in oil type multiple emulsions that are solid or are gelatinized; and multi-phase systems, in particular two-phase systems. These can be made to have the outward appearance of a cream, a salve, a soft paste, an ointment, or a cast or molded solid, particularly a stick. In particular, this can be made into the form of a stick or a dish, and particularly the form of a transparent anhydrous hard gel, and more particularly in the form of a translucent or a transparent anhydrous stick.

These compositions may have uses in particular as body health compositions in the form of, for example, deodorant sticks; hair compositions in the form of, for example, styling sticks or makeup sticks for the hair; makeup compositions for skin or mucous membranes of the face or the body, for example lipstick, foundations cast as a stick or a dish, face powders, eyeshadow, bases for coating onto conventional lipstick, concealer sticks, lip gloss, eyeliner, mascara, or temporary tattoos; compositions for the care of skin or mucous membranes, in the form of, for example, lip care balm or base, body ointments or daily care cream; as sunscreen compositions or self tanning compositions, or skincare compositions, for example, creams or facial cleansing gels.

The spherical optical structure could be present into the cosmetic composition in a quantity of from 0.001% to 74% by weight, preferably from 0.1% to 60% by weight, more preferably from 1% to 30% by weight, relative to the total weight of the composition.

The composition could additionally comprise additives such as oils, waxes, pigments, fillers, dyes, surfactants, water, solvents, alcohols, or mixture thereof.

[Embodiments]

Herebelow, the present invention shall be explained in further detail, giving concrete examples.

(Embodiment 1)

50 ml of 4 to 8 wt% aqueous gelatin solution is emulsified in 200 ml of methylene chloride solution containing 5 wt% of polystyrene (molecular weight 200,000), to prepare a water in oil type primary emulsion (1).

Separately from the above, 1500 ml of a 1 wt% aqueous gelatin solution was prepared, and the abovementioned emulsion (1) was added under agitation, to obtain a water/oil/water type secondary emulsion.

By maintaining the temperature of the system at approximately 40 degrees C, and evaporating the methylene chloride, spherical optical structures having a polystyrene shell and an aqueous solution as the core substance were obtained.

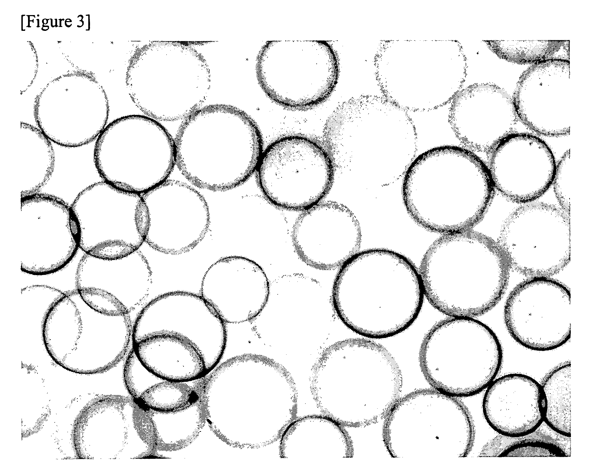

An optical photomicrograph of the obtained spherical optical structures is shown in figure 3. Each spherical optical structure displayed a structural color according to the thickness of its shell.

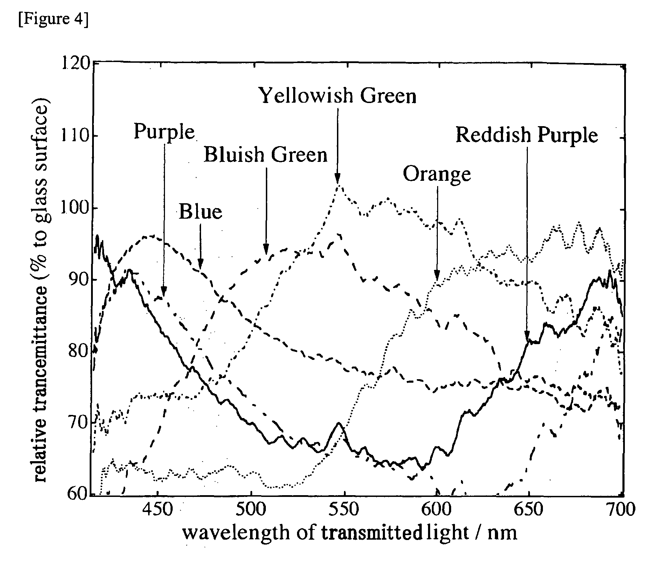

The transmission spectrum of spherical optical structures of each color obtained is shown in figure 4. It was confirmed that light transmitted by each of the spherical optical structures had a wavelength peak of transmitted light corresponding to each color.

[Brief Description of the Drawings]

[Figure 1] Cross sectional geometrical diagram for explaining optical principles of spherical optical structure displaying structural colors of the present invention.

[Figure 2] Schematic diagram showing cross section of spherical optical structure displaying red and blue structural color of the present invention.

[Figure 3] Optical photomicrograph of spherical optical structures obtained in embodiment 1.

[Figure 4] Comparison of transmission spectra of spherical optical structures of each color obtained in embodiment 1.

[Explanation of Reference Numbers]

| | |
|---|---|
| 10 | Spherical optical structure |
| 11 | Coating |
| 12 | Core |
| 13 | External medium |
| 14 | Light beam |
| D | Coating thickness |
| L | Particle radius of spherical structure |
| O | Center of spherical optical structure |
| P | Point of incidence of light beam onto coating |
| Q | Midpoint between point of incidence onto coating and point of exit from coating of light beam |
| R | Point of reflection of light beam at coating inner wall S2 |
| S 1 | Coating outer wall |
| S2 | Coating inner wall |
| T | Point of S1 on line extended from OQ |
| U | Point of exit of light beam from coating |
| d | Distance between point T and point Q |
| 20 | Red spherical optical structure |
| 30 | Blue spherical optical structure |
| 31 | Core |
| 32 | Coating |

**Claims**

1. A spherical optical structure, **characterized by** being a spherical optical structure having a particle diameter of 500 $\mu$m or less, comprising a core substance and a coating substance coating the core substance, wherein the particle diameter and the thickness of the coating substance of said spherical optical structure are controlled to be in a predetermined range, according to the refractive index of an external medium in contact with the outer surface of said spherical optical structure and the refractive index of said coating substance, so as to display a structural color in the visible light range.

2. A spherical optical structure in accordance with Claim 1, wherein the particle radius L and the thickness of the coating substance D of said spherical optical structure are controlled, according to the refractive index $n_1$ of the external medium in contact with the outer surface of said spherical optical structure and the refractive index $n_2$ of said coating substance, to display a structural color having a wavelength $\lambda$ in the visible light range obtained by substituting a value d satisfying

[Equation 1]

$$sin\{90 - \arccos(\frac{L-d}{L})\} = \frac{n_2}{n_1} sin[\{90 - \arccos(\frac{L-d}{L})\} - \arctan[\frac{D-d}{Lsin\{\arccos(\frac{L-d}{L})\}}]]$$

into

[Equation 2]

$$\frac{\lambda}{2}(2m+1) \quad (m = 0, 1, 2, 3, .....) = \frac{2Lsin\{\arccos(\frac{L-d}{L})\}}{\cos[\arctan[\frac{D-d}{Lsin\{\arccos(\frac{L-d}{L})\}}]]} - 2Lsin\{\arccos(\frac{L-d}{L})\}.$$

(where m is 0 or a natural number).

3. A spherical optical structure in accordance with Claim 1, **characterized in that** the thickness of the coating substance is in the range of 50 to 700 nm.

4. A spherical optical structure in accordance with Claim 1, **characterized in that** the thickness of the coating substance is in the range of 80 to 500 nm.

5. A spherical optical structure in accordance with Claim 1, **characterized in that** the thickness of the coating substance is in the range of 100 to 300 nm.

6. A spherical optical structure in accordance with Claim 1, **characterized by** having a particle diameter of 1 to 500 $\mu$m.

7. A spherical optical structure in accordance with Claim 1, **characterized by** having a particle diameter of 10 to 300 $\mu$m.

8. A spherical optical structure in accordance with Claim 1, **characterized in that** the core substance comprises liquid medium or gas medium.

9. A spherical optical structure in accordance with Claim 8, **characterized in that** the liquid medium is selected from water, an hydro-organic solution comprising water and/or C1-C6 alcohol such as ethanol and/or isopropanol; an aqueous liquid substance, an organic liquid substance, an inorganic solvent, or a mixture thereof.

10. A spherical optical structure in accordance with Claim 8, **characterized in that** the liquid medium is selected from water; an hydro-organic solution comprising water and/or C1-C6 alcohol, and gelatin or hydrophilic thickening agents.

11. A spherical optical structure in accordance with Claim 8, **characterized in that** the gas medium is air.

12. A spherical optical structure in accordance with Claim 1, **characterized in that** the coating substance is a substance selected from vinyl based polymers, polycondensation polymers, cellulose based polymers; silicone based polymers; organic polymers; and/or inorganic materials.

13. A spherical optical structure in accordance with Claim 12, **characterized in that** the coating substance is a substance selected polystyrene, polyethylene, C1-C32 alkyle poly(meth)acrylate, methyl poly(meth)acrylate, or nylon.

14. A spherical optical structure in accordance with Claim 1, **characterized by** being provided as dispersion in a liquid medium.

15. A cosmetic coloring material, **characterized by** comprising a spherical optical structure recited in any one of Claims 1 through 14.

**16.** A cosmetic composition, **characterized by** containing the coloring material recited in Claim 15.

[Figure 1]

[Figure 2]

[Figure 3]

[Figure 4]

European Patent
Office

**EUROPEAN SEARCH REPORT**

Application Number

EP 06 25 3170

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PATENT ABSTRACTS OF JAPAN vol. 007, no. 192 (P-218), 23 August 1983 (1983-08-23) & JP 58 090650 A (BANDOO KAGAKU KK), 30 May 1983 (1983-05-30) * abstract * | 1,2, 8-10, 12-16 | INV. A61K8/02 A61K8/11 A61Q1/00 |
| X | PATENT ABSTRACTS OF JAPAN vol. 004, no. 159 (C-030), 6 November 1980 (1980-11-06) & JP 55 105615 A (TANABE SEIYAKU CO LTD), 13 August 1980 (1980-08-13) * abstract * | 1,2, 8-10, 12-16 | |
| X | PATENT ABSTRACTS OF JAPAN vol. 005, no. 136 (C-069), 28 August 1981 (1981-08-28) & JP 56 072011 A (KYOWA HAKKO KOGYO CO LTD), 16 June 1981 (1981-06-16) * abstract * | 1,2, 8-10, 12-16 | |
| X | US 3 609 000 A (SHIZUO MIYANO ET AL) 28 September 1971 (1971-09-28)  * column 2, line 11 - line 64 * claims | 1,2, 8-10, 12-16 | TECHNICAL FIELDS SEARCHED (IPC) A61K |
| A | PATENT ABSTRACTS OF JAPAN vol. 014, no. 318 (C-0738), 9 July 1990 (1990-07-09) & JP 02 110173 A (NIPPON KAPUSERU PROD:KK; others: 02), 23 April 1990 (1990-04-23) * abstract * | | |
| A | WO 2005/011622 A (L'OREAL; DUMOUSSEAUX, CHRISTOPHE; GOTO, TATSUNARI) 10 February 2005 (2005-02-10) | | |

The present search report has been drawn up for all claims

| Place of search | Date of completion of the search | Examiner |
|---|---|---|
| Munich | 4 October 2006 | PALONIEMI LEGLAND, R |

EPO FORM 1503 03.82 (P04C01)

## ANNEX TO THE EUROPEAN SEARCH REPORT
## ON EUROPEAN PATENT APPLICATION NO.

EP 06 25 3170

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2006

| Patent document cited in search report | | Publication date | Patent family member(s) | | Publication date |
|---|---|---|---|---|---|
| JP 58090650 | A | 30-05-1983 | NONE | | |
| JP 55105615 | A | 13-08-1980 | JP | 1237517 C | 31-10-1984 |
| | | | JP | 59010642 B | 10-03-1984 |
| JP 56072011 | A | 16-06-1981 | JP | 1015523 B | 17-03-1989 |
| | | | JP | 1532725 C | 24-11-1989 |
| US 3609000 | A | 28-09-1971 | DE | 1946680 A1 | 14-05-1970 |
| | | | FR | 2018203 A5 | 29-05-1970 |
| | | | GB | 1281042 A | 12-07-1972 |
| JP 02110173 | A | 23-04-1990 | NONE | | |
| WO 2005011622 | A | 10-02-2005 | EP | 1660024 A1 | 31-05-2006 |
| | | | JP | 2005053846 A | 03-03-2005 |

EPO FORM P0459

For more details about this annex : see Official Journal of the European Patent Office, No. 12/82

## REFERENCES CITED IN THE DESCRIPTION

**Patent documents cited in the description**

- JP 2004526558 A

**Non-patent literature cited in the description**

- Development of Advanced Cosmetics. CMC Publishing Co., Ltd, 2000, 297-306
- **TAMOTSU KONDOU et al.** Microcapsules: Their Production, Properties, and Applications. Sankyou Publishing